# DEMANDE DE BREVET EUROPEEN

(11) **EP 4 537 868 A1**
(43) Date de publication de la demande: **16.04.2025**
(21) Numéro de dépôt: 23202971.0
(22) Date de dépôt: 11.10.2023
(51) Int. Cl.: A61M 5/00

(54) **OUTIL À MAIN DE MANIPULATION DE SERINGUES DE SUBSTANCE PHARMACEUTIQUE OU COSMÉTIQUE**

(71) Demandeur: Cilyx, 4102 Seraing (BE)
(72) Inventeur: MARON, Olivier, 4102 Seraing (BE)
(74) Mandataire: Calysta NV

(57) **Abrégé**

Outil à main de manipulation de seringues comprenant un socle de réception (9) d'un nest (1) pour seringues (2) et un peigne mobile (15), ledit socle (9) comprenant une base (11), une plaque d'accueil (13) pour un nest (1) et une plaque supérieure (14), déplaçable verticalement entre une position haute et une position basse ; et ledit peigne mobile (15) comprend des espaces interdentaires (21) agencés pour loger des seringues (2).

## Description

La présente invention se rapporte à un outil à main de manipulation de seringues de substance pharmaceutique ou cosmétique.

Les substances pharmaceutiques ou cosmétiques sont typiquement conditionnées en seringues. Celles-ci sont livrées en nest aux acteurs de l'industrie pharmaceutique ou cosmétique, dans un boîtier (Tub) de protection qui protège les seringues de la casse, les seringues étant disposées parallèlement et maintenues substantiellement écartées les unes des autres par le nest. Le nest permet également de les maintenir dans l'orientation voulue.

Les nests sont des présentoirs pour seringues et sont munis d'une plaque basale munie moyens de retenue ou de cols dans lesquels les seringues sont enfoncées. Au moment de la livraison, dans le nest se trouvent les corps de seringues à remplir de la substance pharmaceutique ou cosmétique. Le corps de seringue comporte une collerette.

Ensuite, la seringue est munie de son aiguille avec un bouchon et une fois remplie, la seringue sera ensuite pourvue d'un bouchon et d'un piston.

De la livraison des corps de seringues jusqu'au conditionnement final des seringues, après remplissage, de nombreuses manipulations de ces seringues sont réalisées. Ces manipulations sont réalisées généralement par des bras robotisés munis de pinces ou de préhenseurs qui fonctionnent de différentes manières. Les pinces/préhenseurs conventionnels fonctionnent pneumatiquement et sont pourvues de petites ventouses qui permettent la prise des seringues. Ces bras robotisés permettent de fonctionner à haute cadence, typiquement ils permettent la manipulation de plus de 300 conteneurs/min, parfois 600 conteneurs/min.

Un enjeu fréquent de la manipulation des seringues est la modification du positionnement en matrice dans le nest au convoyage en ligne ou inversement. Cet enjeu se présente au moment de la livraison des corps de seringues vides ou au moment de la manipulation de seringues pré-remplies, munies typiquement de leurs bouchons, mais pas nécessairement. Typiquement, lorsque les seringues sont pré-remplies dans une ligne de remplissage, elles ne sont pas encore munies de leur piston.

C'est d'ailleurs dans ce contexte que les pinces robotisées permettent d'enlever une rangée de seringues du nest et placent les conteneurs en ligne, les uns derrières les autres sur le convoyeur.

Si les bras robotisés sont aujourd'hui très bien développés et fréquemment utilisés, ceux-ci ne trouvent une réelle utilité que lorsque la cadence est importante. En effet, dans le cas de production de petites séries ou de petits lots, ou encore dans le cas de convoyeurs plus lents comme ceux qui permettent l'inspection des conteneurs avant utilisation ou placement du piston, par une mireuse surveillée par un opérateur, l'utilisation d'un bras robotisé n'est pas justifiée et le coût de l'automatisation n'est pas absorbé sur la taille de la série. Parfois aussi, la cadence requise ne permet pas l'utilisation d'un bras robotisé, celui-ci n'étant pas prévu pour travailler à faible cadence.

Dans ce contexte, il existe des pinces manuelles que l'opérateur prend en main, qu'il positionne en entrée ou en sortie de convoyeur et qui permettent soit de collecteur une rangée de seringues en sortie de convoyeur et de la placer dans un nest, l'opération étant sans cesse répétée jusqu'au remplissage du nest, soit de collecter une rangée de seringues du nest et de la placer dans un convoyeur, l'opération étant réalisée jusqu'à ce que le nest soit vide.

Malheureusement, cette étape doit être répétée un grand nombre de fois sur la taille d'une petite série, ce qui crée une grande pénibilité du travail, demande un temps considérable pour le renestage (remise en nest des seringues) ou le dénestage (retrait du nest des seringues) ainsi que des risques de casse.

L'invention a pour but de pallier les inconvénients de l'état de la technique en procurant un dispositif de manipulation de seringues qui permet de réduire la pénibilité de l'opération de manipulation manuelle, d'augmenter la vitesse ainsi que de limiter le risque de casse.

Par les termes « seringues », on entend selon la présente invention un corps de seringue, plus particulièrement en verre, munie ou à munir d'une aiguille et d'un capuchon muni en partie supérieure d'une collerette ou un tube pour injecteur sans aiguille pour autant qu'il soit muni en partie supérieure d'une collerette.

Pour résoudre ce problème, il est prévu suivant l'invention un outil à main de manipulation de seringues comprenant un socle de réception d'un nest pour seringues et un peigne mobile,
- ledit socle comprenant une extrémité distale et une extrémité proximale, une base et au moins deux éléments latéraux, une plaque d'accueil pour un nest pour seringues, agencée sensiblement parallèlement à ladite base et à une hauteur h2 mesurée entre une face supérieure de ladite base et une face supérieure de ladite plaque d'accueil, et une plaque supérieure, déplaçable verticalement entre une position haute et une position basse, ladite position basse étant une position dans laquelle une face inférieure de ladite plaque supérieure est espacée de la face supérieure de la plaque d'accueil d'une distance h3 comprise entre 0,5 cm et 5 cm, de préférence entre 1 et 4 cm, plus particulièrement entre 2 et 3 cm, ladite position haute étant une position dans laquelle la distance h3 est comprise entre 6 et 15 cm, de préférence entre 8 et 12 cm, plus particulièrement entre 9.5 et 10,5 cm,
- ledit peigne mobile étant muni d'une base de peigne mobile comprenant une première partie latérale et une deuxième partie latérale et une poignée, et, entre ladite première partie latérale et ladite deuxième partie latérale, x-1 dents de séparation s'étendant d'une partie distale de ladite base et x espaces interdentaires, lesdites dents de séparation et lesdits espaces interdentaires étant orientés de manière sensiblement parallèle l'un à l'autre, lesdits espaces interdentaires étant agencés pour loger des seringues, de préférence n seringues, dans une position selon laquelle des collerettes de corps de seringues reposent sur une face supérieure desdites dents de séparation, ladite première partie latérale et ladite deuxième partie latérale présentant chacune une face inférieure et une face supérieure, ladite face inférieure étant agencée pour coopérer avec ladite face supérieure de la plaque supérieure dudit socle de manière à permettre le coulissement du peigne mobile sur la plaque supérieure dudit socle.

Comme on peut le constater, l'outil de manipulation comprend un socle de réception d'un nest qui comprend une base, une extrémité distale et une extrémité proximale et deux éléments latéraux. Les éléments latéraux peuvent être sous forme de paroi, peine ou non, d'une série d'au moins deux piliers. Les éléments latéraux s'étendent vers le haut à partir de la base ou bien sont positionnés le long de la partie latérale de la base, sensiblement verticalement.

Parallèlement à la base du socle, une plaque d'accueil de nest est positionnée. Celle-ci comprend un orifice ou une cavité dans lequel le nest (ou un nest dans un tub) est logé de manière à ce que les rebords du nest reposent sur une face supérieure de la plaque d'accueil de nest.

Par les termes « rebords du nest reposant sur une face supérieure de la plaque d'accueil du nest », on entend au sens de la présente invention que les rebords du nest reposent directement sur une face supérieure de la plaque d'accueil du nest ou que les rebords du nest reposent sur un rebord du tub et que le rebord du tub repose sur une face supérieure de la plaque d'accueil du nest.

La poignée est disposée au-dessus de la base du peigne mobile, reliée par des moyens de connexion à l'un des éléments parmi la base de peigne mobile, ladite première partie latérale et ladite deuxième partie latérale ou lesdits moyens de coulissement mutuels

La plaque d'accueil du nest est positionnée à une hauteur h2 mesurée entre une face supérieure de ladite base et une face supérieure de ladite plaque d'accueil. Typiquement, h2 est comprise entre 6 et 30.5 cm, plus particulièrement entre 8 et 12 encore plus préférentiellement entre 10 et 11 cm.

Le socle comporte par ailleurs une plaque supérieure, déplaçable verticalement entre une position haute et une position basse, ladite position basse étant une position dans laquelle une face inférieure de ladite plaque supérieure est espacée de la face supérieure de la plaque d'accueil d'une distance h3 comprise entre 0,5 cm et 5 cm, de préférence entre 1 et 4 cm, plus particulièrement entre 2 et 3 cm, ladite position haute étant une position dans laquelle la distance h3 est comprise entre 6 et 15 cm, de préférence entre 8 et 12 cm, plus particulièrement entre 9.5 et 10,5 cm.

Dès plus, ladite position basse est une position dans laquelle une face supérieure de ladite plaque supérieure est espacée de la face supérieure de la base du socle d'une distance h1 qui est la somme de h2 et h3 et qui vaut typiquement au plus la hauteur d'une seringue mesurée sous la collerette diminuée de l'épaisseur du peigne mobile.

L'outil de manipulation de seringue comporte également un peigne mobile muni d'une base de peigne mobile comprenant une première partie latérale et une deuxième partie latérale et une poignée, et, entre ladite première partie latérale et ladite deuxième partie latérale, x-1 dents de séparation s'étendant d'une partie distale de ladite base et x espaces interdentaires, lesdites dents de séparation et lesdits espaces interdentaires étant orientés de manière sensiblement parallèle l'un à l'autre, lesdits espaces interdentaires étant agencés pour loger des seringues, de préférence n seringues, dans une position selon laquelle des collerettes de corps de seringues reposent sur une face supérieure desdites dents de séparation.

Le peigne mobile comprend donc une série de x-1 dents de séparation entre deux parties latérales situées de part et d'autre des dents de séparation qui forment ensemble x espaces interdentaires. Ces x espaces interdentaires sont prévus pour accueillir chacun une série de n seringues, présentant ainsi chacun une longueur suffisante pour accueillir ladite série. En d'autres mots, la longueur de chaque espace interdentaire est supérieure au diamètre des seringues multiplié par le nombre de seringues, soit n.

Les dents de séparation du bloc peigne permettent ainsi de séparer les séries de x seringues contenues dans les espaces interdentaires les unes des autres de telle façon que la distance qui sépare les séries de x seringues logées dans les espaces interdentaires soit sensiblement la même que celle qui sépare les séries de x seringues logées dans un nest.

La première partie latérale et ladite deuxième partie latérale du peigne mobile présentent chacune une face inférieure et une face supérieure, ladite face inférieure étant agencée pour coopérer avec ladite face supérieure de la plaque supérieure dudit socle de manière à permettre le coulissement du peigne mobile sur la plaque supérieure dudit socle.

La hauteur h1 telle qu'indiquée ci-avant correspond au plus, selon les usages, lorsque la plaque supérieure est en position basse à la hauteur sous la collerette d'une seringue munie ou non de son aiguille et de son capuchon de laquelle l'épaisseur des dents du peigne mobile est déduite ainsi que l'épaisseur de la plaque supérieure et éventuellement aussi l'épaisseur de la plaque d'accueil.

De cette manière, le peigne mobile peut être glissé sous les collerettes des seringues lorsque la plaque supérieure est en position basse, pour loger ou déloger les seringues du nest dans les espaces interdentaires du peigne, avec les collerettes qui reposent sur les dents adjacentes des espaces interdentaires.

La hauteur h3 telle qu'indiquée ci-avant, correspond au moins, selon les usages, à la hauteur sous collerette de la seringue, munie ou non de son aiguille et de son capuchon, déduite de l'épaisseur des dents du peigne, ce qui permet de retirer les seringues du socle, lorsque la plaque supérieure est en position haute, en les faisant passer au-dessus du col du nest ou de les amener dans le socle, logée dans le peigne, en les faisant passer au-dessus du col du nest.

Avantageusement, le socle comprend des moyens de levage de ladite plaque supérieure, comme par exemple un mécanisme en ciseaux, un système à un ou plusieurs verrins, ou encore un système de câble(s) et de poulie(s).

Dans un mode de réalisation préféré de la présente invention, lesdits deux éléments latéraux dudit socle comprennent une première paroi latérale et une deuxième paroi latérale, pleine ou non, placée entre ladite base et ladite plaque d'accueil de nest, ainsi qu'un premier pilier et un deuxième pilier, placé entre ladite plaque d'accueil de nest et ladite plaque supérieure, au niveau de l'extrémité distale dudit socle.

Plus particulièrement, selon la présente invention, lesdits deux éléments latéraux dudit socle comprennent en outre un troisième pilier et un quatrième pilier, placé entre ladite plaque d'accueil de nest et ladite plaque supérieure, au niveau de l'extrémité proximale dudit socle. Ce mode de réalisation est particulièrement préféré lorsque les moyens de levage sont de type système de verrins ou de câbles et poulies.

Dans un mode de réalisation particulier selon la présente invention, lesdits deux éléments latéraux dudit socle comprennent une première paroi latérale et une deuxième paroi latérale, pleine ou non, placée entre ladite base et ladite plaque d'accueil de nest, ainsi qu'un premier pilier et un deuxième pilier, placé entre ladite plaque d'accueil de nest et ladite plaque supérieure, au niveau de l'extrémité distale dudit socle et les moyens de levage comprennent deux mécanismes en ciseaux de part et d'autre du socle, chaque mécanisme en ciseaux comportant deux bras de ciseaux montés de manière pivotante autour d'un point de pivot sensiblement central, le premier bras de ciseaux étant relié à une extrémité, à un coin de la plaque supérieure de manière pivotante et à l'autre extrémité à la base dudit socle, du côté de l'extrémité proximale de celui-ci, de manière coulissante via un premier moyen de coulissement, le deuxième bras de ciseaux étant relié à une extrémité à la base dudit socle, du côté de l'extrémité distale, de manière pivotante et à l'autre extrémité, à la plaque supérieure, du côté de l'extrémité proximale du socle, de manière coulissante via un deuxième moyen de coulissement.

Plus particulièrement, selon la présente invention, le premier moyen de coulissement comprend une gorge placée sur ladite base dans laquelle un élément mobile relié au premier bras de ciseau se déplace, ou un rail muni d'orifices sous forme de trous ou d'encoches, placé sur ladite base dans lequel un élément mobile comportant une protubérance complémentaire auxdits orifices, relié au premier bras de ciseau se déplace.

Plus particulièrement, selon la présente invention, le deuxième moyen de coulissement comprend une gorge placée sur ladite plaque supérieure dans laquelle un élément mobile relié au deuxième bras de ciseau se déplace, ou un rail muni d'orifices sous forme de trous ou d'encoches, placé sur ladite plaque supérieure dans lequel un élément mobile comportant une protubérance complémentaire auxdits orifices, relié au deuxième bras de ciseau se déplace.

Dans un mode de réalisation particulier selon la présente invention, la plaque supérieure comporte latéralement, de chaque côté, un épaulement longitudinal comprenant une paroi horizontale et une paroi s'étendant vers le haut, à partir de ladite paroi horizontale, agencé pour guider le peigne mobile pendant son déplacement, et éventuellement un épaulement transversal au niveau de l'extrémité distale de la plaque supérieure, comprenant une paroi horizontale et une paroi s'étendant vers le haut, à partir de ladite paroi horizontale, agencée pour recevoir en butée ledit peigne mobile pendant son déplacement.

Dans un mode de réalisation préférentiel selon la présente invention, ladite plaque supérieure comprend des moyens de centrage dudit peigne mobile, comme par exemple, deux bavettes latérales et une bavette distale.

Dans un autre mode de réalisation avantageux de la présente invention, ledit peigne mobile comprend des moyens d'obturation desdits x espaces interdentaires, situé au niveau de l'extrémité proximale dudit peigne mobile, lesdits moyens d'obturation présentant une position d'ouverture selon laquelle lesdits espaces interdentaires sont accessibles et une position de fermeture selon laquelle lesdits espaces interdentaires sont fermés.

Avantageusement, selon la présente invention, ledit peigne mobile comprend des moyens d'actionnement desdits moyens d'obturation, agencés pour permettre le passage desdits moyens d'obturation de la position d'ouverture à la position de fermeture, ou de la position de fermeture à la position d'ouverture, éventuellement positionnés sur la poignée.

Dans encore un autre mode de réalisation avantageux de la présente invention, ledit peigne mobile comprend des espaces interdentaires pairs agencés pour loger chacun une série de n seringues et former des rangées paires de n seringues et des espaces interdentaires impairs agencés pour loger chacun une série de n seringues et former des rangées impaires de n seringues, et comprend des moyens de centrage agencés pour décaler les rangées paires de n seringues des rangées impaires de n seringues.

L'outil de manipulation de seringues selon la présente invention comprend ainsi un socle et un peigne mobile sur lequel les moyens de centrage peuvent être clipsé lorsque c'est nécessaire ou peut comprendre un premier peigne mobile et un deuxième peigne mobile, l'un comportant alors les moyens de centrage.

Plus particulièrement, selon la présente invention, les moyens de centrage comprennent
- une première plaque dans laquelle sont présents des orifices longitudinaux orientés dans une direction parallèle aux dents du peigne mobile, lesdits orifices longitudinaux surplombant les espaces interdentaires pairs, ladite première plaque étant positionnée au-dessus des dents du peigne mobile et,
- une deuxième plaque positionnée au-dessus de ladite première plaque lorsque le peigne mobile est en position avec la poignée vers le haut et comprenant des saillies longitudinales selon une direction parallèle aux dents du peigne mobile, lesdites saillies longitudinales étant agencées pour passer au travers desdits orifices longitudinaux de la première plaque et de longueur inférieure à la longueur desdits orifices longitudinaux et comprenant sous lesdites saillies, des éléments de reliefs plus particulièrement n éléments de reliefs s'étendant de ladite deuxième plaque vers le bas, lesdits éléments de relief surplombant les espaces interdentaires impairs et étant agencés pour engager les orifices des corps des seringues présentes dans les espaces interdentaires impairs,
- des moyens de commande permettant un déplacement de ladite deuxième plaque par rapport à ladite première plaque lors duquel lesdites saillies longitudinales de la deuxième plaque effectue un déplacement longitudinal dans les orifices longitudinaux de la première plaque, ledit déplacement longitudinal permettant de déplacer les n seringues présentes dans les espaces interdentaires pairs par rapport aux n seringues présentes dans les espaces interdentaires impairs dudit peigne mobile.

Dans un autre mode de réalisation avantageux de la présente invention, les moyens de centrage comprennent
- une première plaque dans laquelle sont présents des orifices longitudinaux orientés dans une direction parallèle aux dents du peigne mobile, lesdits orifices longitudinaux surplombant les espaces interdentaires impairs, ladite première plaque étant positionnée au-dessus des dents du peigne mobile et,
- une deuxième plaque positionnée au-dessus de ladite première plaque lorsque le peigne mobile est en position avec la poignée vers le haut et comprenant des saillies longitudinales selon une direction parallèle aux dents du peigne mobile, lesdites saillies longitudinales étant agencées pour passer au travers desdits orifices longitudinaux de la première plaque et de longueur inférieure à la longueur desdits orifices longitudinaux et comprenant sous lesdites saillies, des éléments de reliefs, plus particulièrement n éléments de reliefs s'étendant de ladite deuxième plaque vers le bas, lesdits éléments de relief surplombant les espaces interdentaires pairs et étant agencés pour engager les orifices des corps des seringues présentes dans les espaces interdentaires pairs,
- des moyens de commande permettant un déplacement de ladite deuxième plaque par rapport à ladite première plaque lors duquel lesdites saillies longitudinales de la deuxième plaque effectue un déplacement longitudinal dans les orifices longitudinaux de la première plaque, ledit déplacement longitudinal permettant de déplacer les n seringues présentes dans les espaces interdentaires impairs par rapport aux n seringues présentes dans les espaces interdentaires pairs dudit peigne mobile.

D'autres formes de réalisation de l'outil à main de manipulation de seringues suivant l'invention sont indiquées dans les revendications annexées.

L'invention a aussi pour objet un procédé de dénestage de seringues à l'aide d'un outil à main de manipulation de seringues comprenant les étapes suivantes :
- Une fourniture d'un nest comprenant des orifices agencés pour loger des seringues comprenant chacun un col s'étendant vers le haut, des seringues munies de collerettes de corps de seringues, leurs aiguilles et de leur capuchons d'aiguille, les collerettes de corps de seringues reposant sur l'extrémité supérieure desdits cols,
- Un placement du nest, dans un socle comprenant une plaque d'accueil de nest, dans un orifice de celle-ci avec les rebords du nest qui reposent sur la plaque d'accueil de nest et les capuchons d'aiguilles qui reposent sur une base dudit socle, lesdites collerettes étant en outre relevées et espacées d'une distance hA mesurée entre une face inférieure de ladite collerette et l'arête supérieure dudit col,
- Une préhension d'un peigne mobile comprenant une poignée et x-1 dents de séparation d'une épaisseur hB et x espaces interdentaires, lesdits espaces interdentaires étant agencés pour loger des seringues, de préférence n seringues, dans une position selon laquelle des collerettes de corps de seringues reposent sur une face supérieure desdites dents de séparation,
- Une amenée du peigne mobile par coulissement ou glissement sur une plaque supérieure dudit socle présentant une position basse et une position haute,
- Un premier glissement du peigne mobile sous les collerettes des seringues, durant lequel la plaque supérieure est en position basse et sur laquelle se déplace le peigne mobile, jusqu'à loger dans les espaces interdentaires les seringues,
- Un levage de ladite plaque supérieure jusqu'à sa position haute,
- Un deuxième glissement du peigne mobile dans le sens inverse dudit premier glissement, ledit peigne comportant dans les espaces interdentaires les seringues, et un retrait du peigne mobile agencé pour déplacer les seringues vers une destination voulue.

Dans une forme de réalisation particulière, le procédé de dénestage selon l'invention comprend une obturation des espaces interdentaires qui a lieu entre le levage et le deuxième glissement ou entre le premier glissement et le levage par actionnement de moyens d'obturation des espaces interdentaires.

Dans un autre mode de réalisation avantageux du procédé selon la présente invention, le procédé comprend en outre un positionnement de la plaque supérieure en position basse avant le premier glissement du peigne mobile, ou avant l'amenée du peigne mobile.

D'autres formes de réalisation du procédé de dénestage à l'aide d'un outil à main de manipulation de seringues suivant l'invention sont indiquées dans les revendications annexées.

L'invention a aussi pour objet un procédé de remise en nest de seringues à l'aide d'un outil à main de manipulation de seringues comprenant les étapes de :
- Une fourniture d'un nest ou d'un tub comprenant un nest comprenant des orifices agencés pour loger des seringues comprenant chacun un col s'étendant vers le haut,
- Un placement dudit nest ou tub comprenant le nest dans un socle comprenant une plaque d'accueil de nest, dans un orifice de celle-ci avec les rebords du nest ou du tub qui comprend le nest qui reposent sur la plaque d'accueil de nest,
- Une préhension d'un peigne mobile comprenant une poignée et x-1 dents de séparation et x espaces interdentaires, lesdits espaces interdentaires logeant des seringues, de préférence n seringues, dans une position selon laquelle des collerettes de corps de seringues reposent sur une face supérieure desdites dents de séparation,
- Une amenée du peigne mobile par déplacement sur une plaque supérieure dudit socle présentant une position basse et une position haute,
- Un placement du peigne mobile sur la plaque supérieure en position haute,
- Un passage de ladite plaque supérieure de sa position haute à la position basse,
- Un glissement du peigne mobile vers l'extrémité proximale dudit socle sur ladite plaque supérieure et sous les collerettes ses seringues durant lequel les seringues sont positionnées dans les cols et extraites dudit peigne mobile, et un retrait du peigne mobile.

Dans une forme de réalisation du procédé de remise en nest selon la présente invention, le procédé comprend en outre un positionnement de la plaque supérieure en position haute est effectué avant le premier glissement du peigne mobile, ou avant l'amenée du peigne mobile

Dans encore un autre mode de réalisation du procédé de remise en nest selon la présente invention, les espaces interdentaires sont obturés durant la préhension du peigne mobile et libérés avant le glissement du peigne mobile, comme par exemple durant l'une des étapes parmi l'amenée du peigne mobile, le premier glissement, le passage de ladite plaque supérieure de sa position haute à la position basse ou encore juste avant le glissement, mais après le passage de ladite plaque supérieure de sa position haute à la position basse par actionnement de moyens d'obturation des espaces interdentaires.

Dans un mode préférentiel, le procédé de remise en nest selon la présente invention comprend, préalablement audit glissement, un déplacement des seringues des rangées paires présentes dans des espaces interdentaires pairs par rapport aux seringues des rangées impaires présentes dans des espaces interdentaires impairs, de manière à former des rangées de seringues en quinconce les unes par rapport aux autres.

D'autres formes de réalisation du procédé de remise en nest de seringues suivant l'invention sont indiquées dans les revendications annexées.

D'autres caractéristiques, détails et avantages de l'invention ressortiront de la description donnée ci-après, à titre non limitatif et en faisant référence aux dessins.

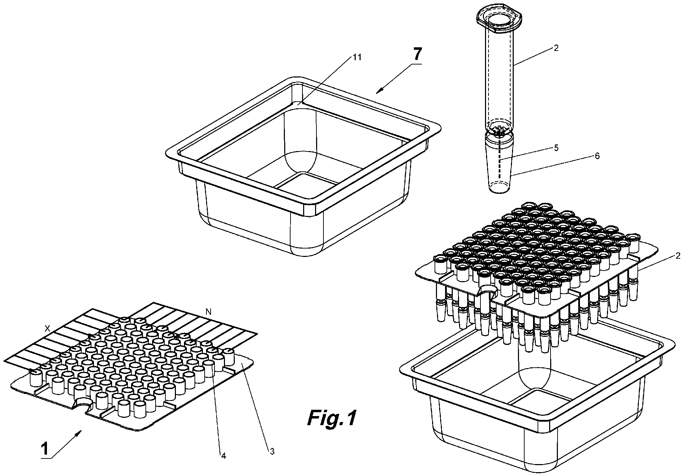

Dans les dessins, la Figure 1 comporte à gauche une vue en perspective d'un nest à seringues, une vue en perspective d'un tub ou boîtier destiné à contenir un nest en haut et une vue éclatée d'un nest contenant des seringues dans un tub ou boîtier à droite.
La figure 2 représente un outil à main de manipulation de seringues selon la présente invention lorsqu'il est utilisé pour extraire des seringues d'un nest où le nest est engagé dans l'outil et le peigne est en position d'approche.
La figure 3 représente un outil à main de manipulation de seringues selon la présente invention lorsqu'il est utilisé pour extraire des seringues d'un nest où le nest est engagé dans l'outil et le peigne initie son engagement dans l'outil.
La figure 4 représente un outil à main de manipulation de seringues selon la présente invention lorsqu'il est utilisé pour extraire des seringues d'un nest où le nest est engagé dans l'outil et où des rangées de seringues sont logées dans le peigne.
La figure 5 est une vue en coupe de la figure 4 selon la ligne de coupe V-V
La figure 6 est une vue en coupe selon la ligne V-V de la figure 4, représentant l'outil à main de manipulation de seringues selon la présente invention où toutes les seringues sont logées dans le peigne.
La figure 7 est une vue en coupe selon la ligne V-V de la figure 4, représentant le retrait du peigne muni des seringues de l'outil à main de manipulation de seringues selon la présente invention.
La figure 8 est une vue en coupe selon la ligne V-V de la figure 4, représentant le peigne muni des seringues retiré de l'outil à main de manipulation de seringues selon la présente invention et où le nest est vidé.
La figure 9 représente un deuxième peigne mobile de l'outil à main de manipulation de seringues selon l'invention.
Les figures 10A et 10B sont des vues de dessous du peigne mobile illustré à la figure 9 représentant les moyens de centrage non activé (figure 10A) et activés (figure 10B).
La figure 11 représente un outil à main de manipulation de seringues selon la présente invention lorsqu'il est utilisé pour remettre en nest des seringues issues d'un convoyeur, montrant l'entrée du tub dans l'outil à main ainsi que l'arrivée du peigne muni des seringues.
La figure 12 représente l'outil à main de manipulation de seringues selon la présente invention où le tub est positionné pour recevoir les seringues en provenance du peigne positionné dans le socle.
La figure 13 représente l'outil à main où la plaque supérieure du socle est en position basse.
La figure 14 est une vue en coupe de la figure 11 selon la ligne XII-XII présentée à la figure 11.
La figure 15 représente le retrait du peigne pendant le renestage des seringues.
La figure 16 est une vue en perspective d'un châssis à relier à un convoyeur d'alimentation de seringues pour faciliter le remplissage du peigne mobile de la figure 9.

Sur les figures, les éléments identiques ou analogues portent les mêmes références

Comme on peut le voir à la figure 1, un nest 1, comme par exemple un nest 1 à seringues 2 est un présentoir muni d'une plaque basale 3 de laquelle s'étendent des moyens de retenue ou cols 4 dans lesquels les seringues 2 sont introduites, munies de leurs aiguilles 5 avec un bouchon 6. Les nests 1 sont typiquement placés dans des boîtiers ou tub 7 qui comprennent un épaulement intérieur 8 sur leur pourtour sur lequel repose la plaque basale 3 du nest 1.

Le nest 1 une fois rempli comprend x séries de n seringues 2 disposées en matrice ou n séries de x seringues 2. On appelle ainsi le nest un nest de x sur n seringues.

La présente invention concerne un outil à main de manipulation de seringues qui permet le retrait des seringues d'un nest (dénestage) ou la remise de seringues (renestage) en nest d'au moins n sur x (n multiplié par x) seringues permettant de réduire la pénibilité de l'opération de renestage manuelle, d'augmenter la vitesse ainsi que de limiter le risque de casse.

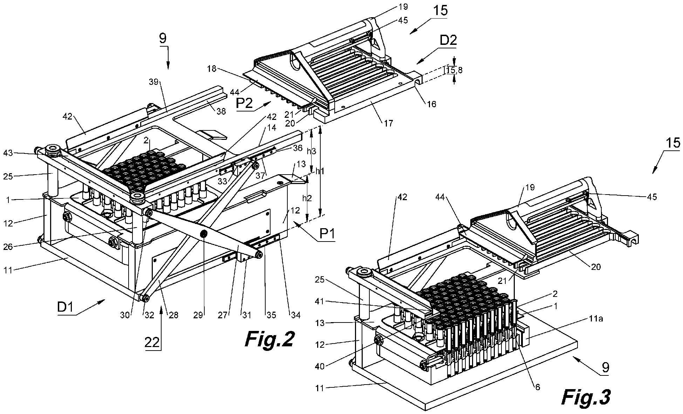

Comme on peut le voir à la figure 2, l'outil à main de manipulation de seringues selon la présente invention comprend un socle 9 de réception d'un nest 1 pour seringues 2 et un peigne mobile 10.

Le socle 9 comprend une extrémité distale D1 et une extrémité proximale P1, une base 11 et au moins deux éléments latéraux 12. Il comprend également une plaque d'accueil 13 pour un nest 1 pour seringues 2 qui est agencée sensiblement parallèlement à ladite base. La plaque d'accueil se situe à une hauteur h2 mesurée entre la face supérieure de ladite base 11 et la face supérieure de ladite plaque d'accueil 13.

Le socle 9 comprend par ailleurs une plaque supérieure 14, déplaçable verticalement entre une position haute et une position basse. A la figure 2, la plaque supérieure 14 est en position haute c'est-à-dire une position dans laquelle la face supérieure de ladite plaque supérieure 14 est espacée de la face supérieure de la plaque d'accueil 13 d'une distance h3 comprise entre 6 et 15 cm, de préférence entre 8 et 12 cm, plus particulièrement entre 9,5 et 10,5 cm et où typiquement, la hauteur h2, mesurée entre la face supérieure de la plaque d'accueil 13 et la face supérieure de la base 11 est comprise entre 6 et 30.5 cm, plus particulièrement entre 8 et 12 encore plus préférentiellement entre 10 et 11 cm, alors que la distance h1, mesurée entre la face supérieure de la plaque supérieure 14 et la face supérieure de la base 11 du socle vaut la somme de h2 et h3 et vaut typiquement au plus la hauteur d'une seringue mesurée sous la collerette diminuée de l'épaisseur du peigne mobile.

L'outil à main illustré à la figure 2 comprend également un peigne mobile 15. Le peigne mobile est muni d'une base 16 de peigne mobile 15 comprenant une première partie latérale 17 et une deuxième partie latérale 18 et une poignée 19.

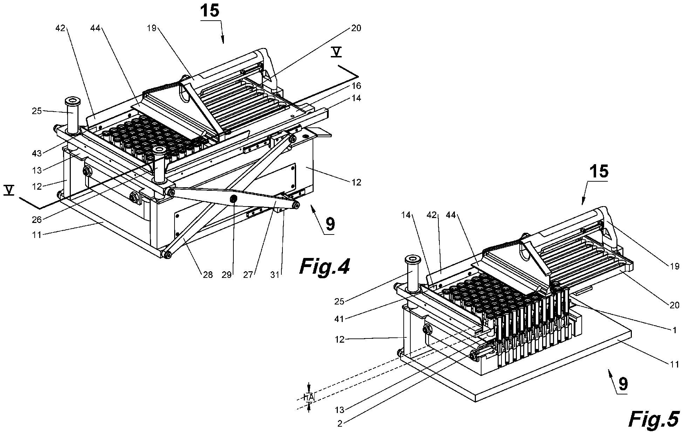

Entre la première partie latérale 17 et la deuxième partie latérale 18, x-1 dents de séparation 20 s'étendent d'une partie distale D2 de ladite base 16 et x espaces interdentaires 21. Les dents de séparation 20 et les espaces interdentaires 21 sont orientés de manière sensiblement parallèle l'un à l'autre. Les espaces interdentaires 21 sont agencés pour loger des seringues 2 (voir par exemple la figure 5), de préférence n seringues, dans une position selon laquelle des collerettes de corps de seringues reposent sur une face supérieure desdites dents de séparation 20.

La première partie latérale 17 et ladite deuxième partie latérale 18 du peigne mobile 15 présentent chacune une face inférieure et une face supérieure, la face inférieure est agencée pour coopérer avec ladite face supérieure de la plaque supérieure 14 dudit socle de manière à permettre le coulissement du peigne mobile 15 sur la plaque supérieure 14 dudit socle 9.

Le socle 9 comprend des moyens de levage 22 de ladite plaque supérieure 14, comme par exemple un mécanisme en ciseaux, un système à un ou plusieurs vérins, ou encore un système de câble(s) et de poulie(s) qui permet le passage de la plaque supérieure de la position haute illustrée à la figure 2 à la position basse illustrée par exemple à la figure 4.

Dans la forme de réalisation illustrée, les deux éléments latéraux 12 dudit socle 9 comprennent une première paroi latérale 23 et une deuxième paroi latérale 24, pleine ou non, placée entre ladite base 11 et ladite plaque d'accueil de nest 13, ainsi qu'un premier pilier 25 et un deuxième pilier 26, placé entre ladite plaque d'accueil de nest 13 et ladite plaque supérieure 14, au niveau de l'extrémité distale D1 dudit socle 9. Les moyens de levage 22 comprennent deux mécanismes en ciseaux de part et d'autre du socle 9, chaque mécanisme en ciseaux comportant deux bras de ciseaux 27, 28 montés de manière pivotante autour d'un point de pivot 29 sensiblement central, le premier bras de ciseaux 27 étant relié à une extrémité, à un coin 30 de la plaque supérieure 14 de manière pivotante et à l'autre extrémité à la base 11 dudit socle 9, du côté de l'extrémité proximale P1 de celui-ci, de manière coulissante via un premier moyen de coulissement.

Le premier moyen de coulissement comprend une gorge 34 placée sur ladite base 11 dans laquelle un élément mobile 31 relié au premier bras de ciseau 27 se déplace, horizontalement, ou un rail 34 muni d'orifices 35 sous forme de trous ou d'encoches, placé sur ladite base 11 dans lequel un élément mobile 31 comportant une protubérance complémentaire auxdits orifices, relié au premier bras de ciseau 27 se déplace horizontalement.

Le deuxième bras de ciseaux 28 étant relié à une extrémité, à un coin 32 de la base 11 dudit socle 9, du côté de l'extrémité distale D1, de manière pivotante et à l'autre extrémité, à la plaque supérieure 14, du côté de l'extrémité proximale P1 du socle, de manière coulissante via un deuxième moyen de coulissement. Le deuxième moyen de coulissement comprend une gorge 36 placée sur ladite plaque supérieure 14 dans laquelle un élément mobile 33 relié au deuxième bras de ciseau 28 se déplace horizontalement, ou un rail 36 muni d'orifices 37 sous forme de trous ou d'encoches, placé sur ladite plaque supérieure 14 dans lequel un élément mobile 33 comportant une protubérance complémentaire auxdits orifices, relié au deuxième bras de ciseau 28 se déplace horizontalement.

La plaque supérieure 14 comporte latéralement, de chaque côté, un épaulement longitudinal comprenant une paroi horizontale 38 et une paroi s'étendant vers le haut 39, à partir de ladite paroi horizontale 38, agencé pour guider le peigne mobile 15 pendant son déplacement, comme cela apparaît par exemple de la figure 3 où on peut voir par ailleurs la présence d'un épaulement transversal au niveau de l'extrémité distale D1 de la plaque supérieure 14, comprenant une paroi horizontale 40 et une paroi s'étendant vers le haut 41, à partir de ladite paroi horizontale 40. La paroi s'étendant vers le haut 41 est agencée pour recevoir en butée ledit peigne mobile 15 pendant son déplacement (horizontal).

La plaque supérieure 14 comprend des moyens de centrage dudit peigne mobile sous forme de deux bavettes latérales 42 et une bavette distale 43. L'extrémité des bavettes latérales 42 ou distale 43 aboutit au niveau de la fin de la paroi s'étendant vers le haut 39 ou 41, respectivement.

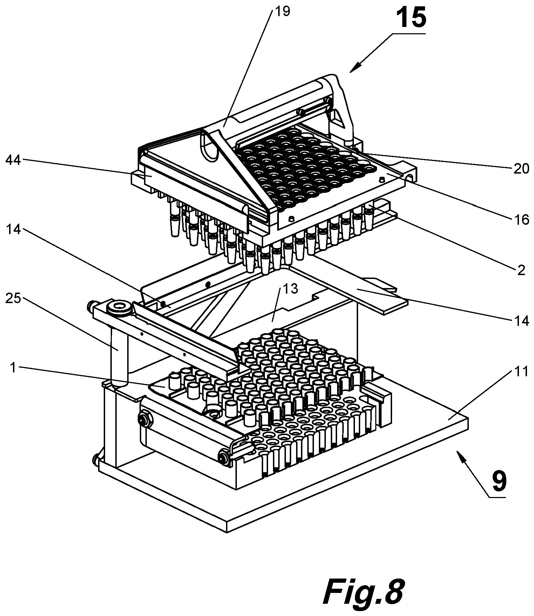

Le peigne mobile 15 comprend des moyens d'obturation 44 desdits x espaces interdentaires 21, situé au niveau de l'extrémité proximale P2 dudit peigne mobile 15. Les moyens d'obturation 44 présentant une position d'ouverture (illustrée à la figure 2) selon laquelle lesdits espaces interdentaires 21 sont accessibles et une position de fermeture selon laquelle lesdits espaces interdentaires 21 sont fermés (voir par exemple la figure 8). Ces moyens d'obturation sont actionnés par des moyens d'actionnement 45, qui permettent le passage desdits moyens d'obturation 44 de la position d'ouverture à la position de fermeture, ou de la position de fermeture à la position d'ouverture, éventuellement positionnés sur la poignée 19.

Les figures 2 à 8 sont des illustrations de la séquence du dénestage des seringues tel que prévu dans le procédé selon la présente invention.

Un nest 1 comprenant des orifices agencés pour loger des seringues 2 comprenant chacun un col s'étendant vers le haut rempli de seringues 2 munies de collerettes de corps de seringues, leurs aiguilles et de leur capuchons d'aiguille avec les collerettes de corps de seringues reposant sur l'extrémité supérieure desdits cols est fourni et placé dans un socle 9 comprenant une plaque d'accueil13 de nest 1, dans un orifice de celle-ci avec les rebords du nest 1 qui reposent sur la plaque d'accueil 13 de nest 1 et les capuchons d'aiguilles qui reposent sur une base 11 dudit socle 9, éventuellement via un bloc 11b pour ajuster la hauteur. Cette étape est illustrée à la figure 2. On peut y voir que les collerettes sont en outre relevées et espacées d'une distance hA mesurée entre une face inférieure de ladite collerette et l'arête supérieure dudit col. La distance hA est supérieure à l'épaisseur hB des dents de séparation 20 du peigne mobile 15.

A la figure 2, le peigne mobile 15, saisi par l'opérateur (non illustré) est amené vers le socle 9.

Ensuite, comme on peut le voir à la figure 3, le peigne est introduit par coulissement ou glissement sur la plaque supérieure 14 dudit socle 9 présentant une position basse et une position haute.

Dans la forme de réalisation illustrée, le peigne est introduit lorsque la plaque supérieure est en position haute, mais il pourrait aussi être introduit lorsque la plaque supérieure est en position basse.

Le premier glissement du peigne mobile sous les collerettes des seringues, durant lequel la plaque supérieure est en position basse et sur laquelle se déplace le peigne mobile, jusqu'à loger dans les espaces interdentaires les seringues est illustré aux figures 4, 5 et 6. On peut y voir que des rangées de seringues sont logées dans le peigne.

Comme on peut le voir à la figure 4, dans le socle 9, la plaque supérieure est en position basse, c'est-à-dire une position dans laquelle la face supérieure de ladite plaque supérieure est espacée de la face supérieure de la base du socle d'une distance h1 comme à la figure 2 et où la plaque supérieure 14 est espacée de la face supérieure de la plaque d'accueil 13 d'une distance h3 comprise entre 0,5 cm et 5 cm, de préférence entre 1 et 4 cm, plus particulièrement entre 2 et 3 cm.

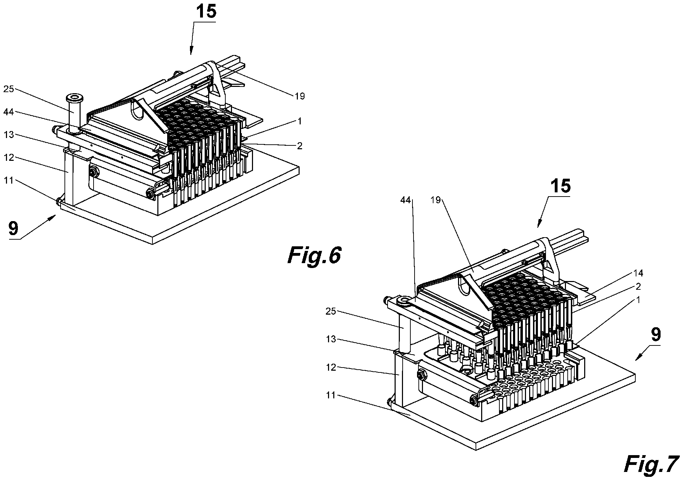

Les espaces interdentaires 21 du peigne mobile 15 sont agencés pour loger des seringues 2, de préférence n seringues 2, dans une position selon laquelle des collerettes de corps de seringues reposent sur une face supérieure desdites dents de séparation 20 du peigne mobile 15. Lorsque toutes les rangées, à savoir les x rangées de n seringues 2 sont logées dans les dents (voir figure 6), l'étape de levage de la plaque supérieure 14 du socle 9 est initiée, jusqu'à ce que la plaque supérieure atteigne sa position haute (voir figure 7).

Ensuite, le procédé selon la présente invention comprend un deuxième glissement du peigne mobile (illustré à la figure 8) dans le sens inverse dudit premier glissement. Le peigne mobile 15 qui comporte dans les espaces interdentaires 21 les seringues 2. Le peigne mobile 15 est ensuite retiré de son socle 9 pour déplacer les seringues 2 vers une destination voulue.

L'outil de manipulation de seringues peut être aussi utilisé dans des opérations de renestage. Dans ce cas, des moyens de centrage sont présents sur le peigne mobile. Ces moyens de centrage peuvent être clipsé sur le peigne mobile décrit pour les figures 2 à 8 ou présents sur un autre peigne mobile de telle façon que l'outil à main comprenne un socle et deux peignes mobiles différents, l'un plus adapté au dénestage et l'autre plus adapté au renestage.

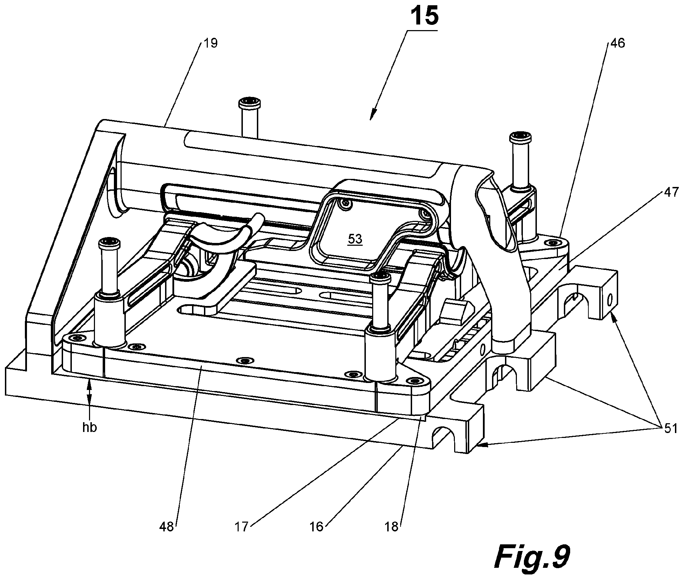

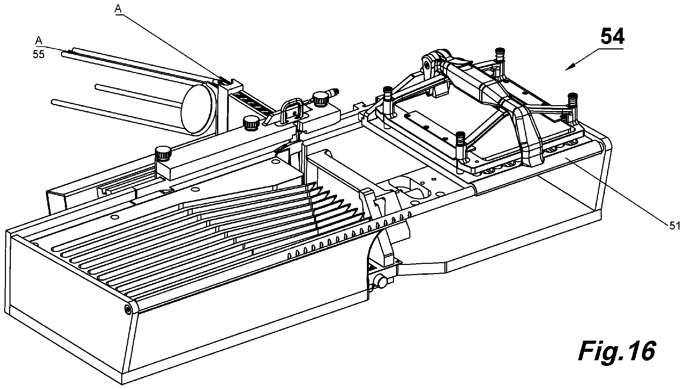

Dans la forme de réalisation illustrée aux figure 9 à 16, l'outil à main selon la présente invention comprend le socle 9 ainsi que le peigne mobile 15 de dénestage et un peigne mobile de renestage.

La figure 9 illustre le peigne de renestage qui comprend des espaces interdentaires pairs agencés pour loger chacun une série de n seringues 2 et former des rangées paires de n seringues 2 et des espaces interdentaires impairs agencés pour loger chacun une série de n seringues 2 et former des rangées impaires de n seringues, et comprend des moyens de centrage 46 agencés pour décaler les rangées paires de n seringues 2des rangées impaires de n seringues 2.

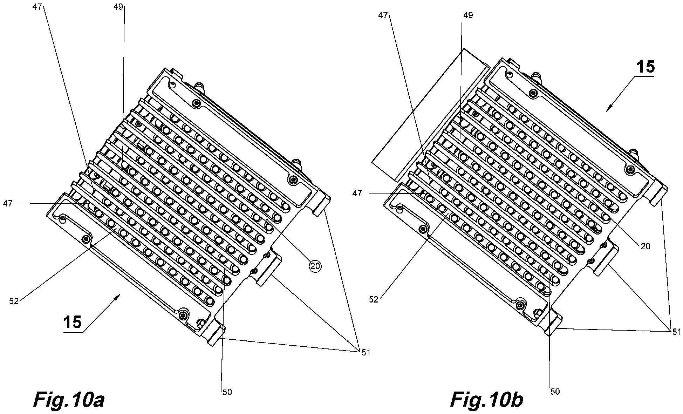

Comme on peut le voir plus en détails à la figure 10A et à la figure 10B, Les moyens de centrage 46 comprennent une première plaque 47 dans laquelle sont présents des orifices longitudinaux 49 orientés dans une direction parallèle aux dents 20 du peigne mobile 15. Les orifices longitudinaux 49 surplombent les espaces interdentaires impairs 21. La première plaque 47 est positionnée au-dessus des dents 20 du peigne mobile 15.

Les moyens de centrage comprennent par ailleurs une deuxième plaque 48 positionnée au-dessus de ladite première plaque 47 lorsque le peigne mobile est en position poignée vers le haut et comprenant des saillies longitudinales 52 selon une direction parallèle aux dents 20 du peigne mobile 15. Les saillies longitudinales 52 sont agencées pour passer au travers desdits orifices longitudinaux 49 de la première plaque 47 et de longueur inférieure à la longueur desdits orifices longitudinaux47. Les saillies longitudinales 52 comprennent des éléments de reliefs 50, plus particulièrement n éléments de reliefs 50 s'étendant de ladite deuxième plaque 48 vers le bas. Les éléments de relief 50 surplombent les espaces interdentaires pairs 21 et sont agencés pour engager les orifices des corps des seringues 2 présentes dans les espaces interdentaires pairs.

Les moyens de centrage comprennent également des moyens de commande 53 (voir figure 9) permettant un déplacement de ladite deuxième plaque 48 par rapport à ladite première plaque 47 lors duquel lesdites saillies longitudinales 52 de la deuxième plaque 48 effectuent un déplacement longitudinal dans les orifices longitudinaux 49 de la première plaque 47, ledit déplacement longitudinal permettant de déplacer les n seringues 2 présentes dans les espaces interdentaires 21 impairs par rapport aux n seringues 2 présentes dans les espaces interdentaires pairs 20 dudit peigne mobile 15.

Comme on peut le comprendre de ce qui précède, les saillies longitudinales ont été décrites comme surplombant les espaces interdentaires pairs, mais il est également possible de positionner les saillies longitudinales de manière qu'elles surplombent les espaces interdentaires impairs.

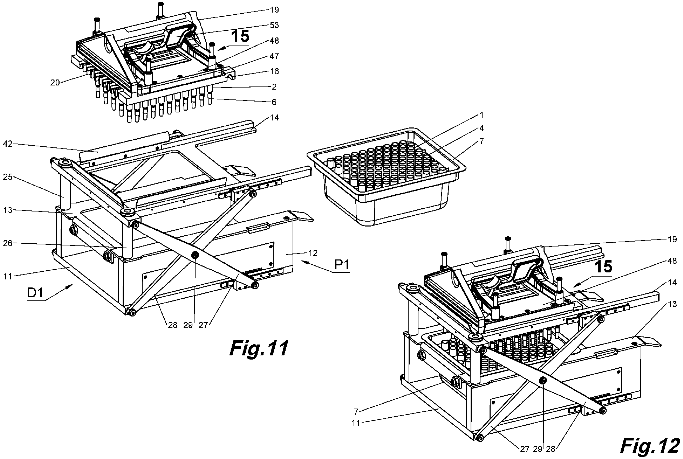

Le procédé de renestage ou de remise en nest est maintenant décrit à l'aide des figures 11 à 15.

Un nest ou un tub 7 (voir figure 11) comprenant un nest 1 est introduit dans la plaqe d'accueil 13 du socle. Le nest 1 comprend des orifices agencés pour loger des seringues 2 comprenant chacun un col 4 s'étendant vers le haut, Le tub 7comprenant le nest 1 est placé dans le socle 9 comprenant une plaque d'accueil 13 de nest 1, dans un orifice de celle-ci avec les rebords du tub 7 qui comprend le nest 1 qui reposent sur la plaque d'accueil 13 de nest 1.

Le peigne mobile 15 comprend x espaces interdentaires 21 logeant n seringues 2 chacun, dans une position selon laquelle des collerettes de corps de seringues 2 reposent sur une face supérieure desdites dents de séparation 20, est amené par déplacement sur une plaque supérieure 14 dudit socle 9 en une position haute.

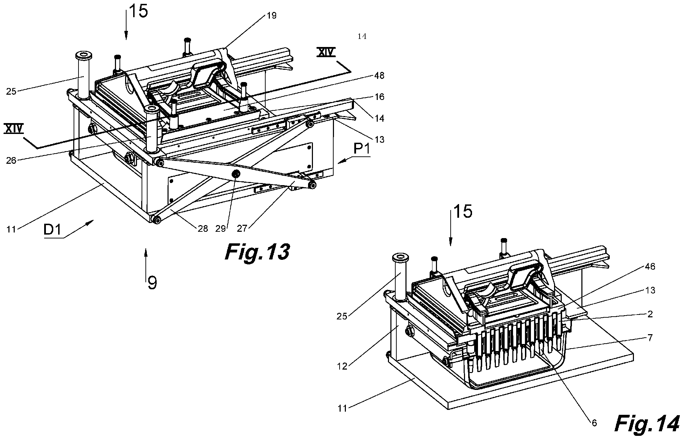

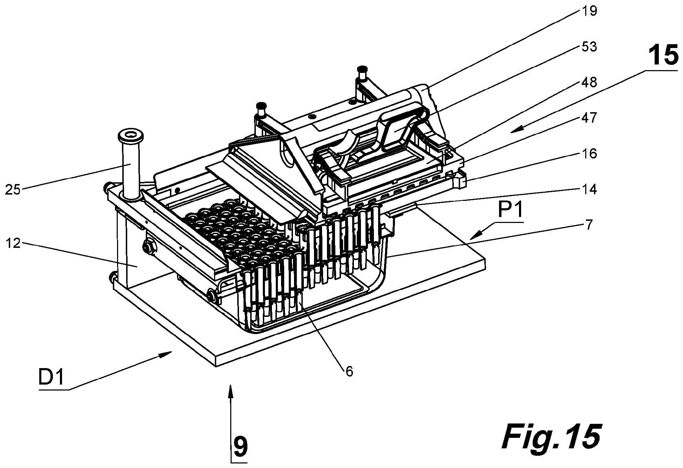

Le peigne mobile 15 muni des seringues 2 est ainsi placé sur la plaque supérieure 14 en position haute (voir figure 12), et la plaque supérieure 14 passe de sa position haute à la position basse (voir figure 13 et figure 14). Ensuite, le peigne mobile 15 est glissé vers l'extrémité proximale P1 (voir figure 15) dudit socle 9 sur ladite plaque supérieure 14 et sous les collerettes ses seringues 2 durant lequel les seringues sont positionnées dans les cols 4 et extraites dudit peigne mobile 15 avant d'être retiré.

Dans une forme de réalisation préférentielle, l'outil à main de manipulation de seringues comprend également un châssis 54 à relier à un convoyeur d'alimentation 55 de seringues qui permet la remise des seringues en lignes en position de matrice pour le remplissage du peigne de renestage mobile 15. Il comprend des moyens de coulissement sur lesquels des moyens de coulissement 51 du peigne mobile 15 peuvent coulisser.

Il est bien entendu que la présente invention n'est en aucune façon limitée aux formes de réalisations décrites ci-dessus et que bien des modifications peuvent y être apportées sans sortir du cadre des revendications annexées.

Par exemple, il est envisagé au sens de la présente invention que les deux éléments latéraux comprennent un troisième pilier et un quatrième pilier, placé entre ladite plaque d'accueil de nest et ladite plaque supérieure, au niveau de l'extrémité proximale dudit socle et que la plaque supérieure coulisse verticalement le long des premier, deuxième, troisième et quatrième piliers, par un système de câbles et de poulies, de vérins ou encore par des tubes télescopiques, motorisés ou non.

## Revendications

1. Outil à main de manipulation de seringues comprenant un socle de réception (9) d'un nest (1) pour seringues (2) et un peigne mobile (15),
- ledit socle (9) comprenant une extrémité distale (D1) et une extrémité proximale (P1), une base (11) et au moins deux éléments latéraux (12), une plaque d'accueil (13) pour un nest (1) pour seringues (2), agencée sensiblement parallèlement à ladite base (11) et à une hauteur h1 mesurée entre une face supérieure de ladite base (11) et une face supérieure de ladite plaque d'accueil (13), et une plaque supérieure (14), déplaçable verticalement entre une position haute et une position basse, ladite position basse étant une position dans laquelle une face inférieure de ladite plaque supérieure est espacée de la face supérieure de la plaque d'accueil d'une distance h3 comprise entre 0,5 cm et 5 cm, de préférence entre 1 et 4 cm, plus particulièrement entre 2 et 3 cm, ladite position haute étant une position dans laquelle la distance h3 est comprise entre 6 et 15 cm, de préférence entre 8 et 12 cm, plus particulièrement entre 9.5 et 10,5 cm,
- ledit peigne mobile (15) étant muni d'une base (11) de peigne mobile (15) comprenant une première partie latérale (17) et une deuxième partie latérale (18) et une poignée (19), et, entre ladite première partie latérale (17) et ladite deuxième partie latérale (18), x-1 dents de séparation (20) s'étendant d'une partie distale (D2) de ladite base (16) et x espaces interdentaires (21), lesdites dents de séparation (20) et lesdits espaces interdentaires (21) étant orientés de manière sensiblement parallèle l'un à l'autre, lesdits espaces interdentaires (21) étant agencés pour loger des seringues (2), de préférence n seringues (2), dans une position selon laquelle des collerettes de corps de seringues (2) reposent sur une face supérieure desdites dents de séparation (20), ladite première partie latérale (17) et ladite deuxième partie latérale (18) présentant chacune une face inférieure et une face supérieure, ladite face inférieure étant agencée pour coopérer avec ladite face supérieure de la plaque supérieure (14) dudit socle (9) de manière à permettre le coulissement du peigne mobile (15) sur la plaque supérieure dudit socle (9).

2. Outil à main de manipulation de seringues selon la revendication 1, dans lequel le socle (9) comprend des moyens de levage (22) de ladite plaque supérieure (14), par exemple un mécanisme en ciseaux, un système à un ou plusieurs verrins, ou encore un système de câble(s) et de poulie(s).

3. Outil à main de manipulation de seringues selon la revendication 1 ou 2, dans lequel lesdits deux éléments latéraux (12) dudit socle (9) comprennent une première paroi latérale (12) et une deuxième paroi latérale (12), pleine ou non, placée entre ladite base (11) et ladite plaque d'accueil (13) de nest, ainsi qu'un premier pilier (25) et un deuxième pilier (26), placé entre ladite plaque d'accueil (13) de nest (1) et ladite plaque supérieure (14), au niveau de l'extrémité distale (D1) dudit socle (9) et où, éventuellement lesdits deux éléments latéraux (12) dudit socle (9) comprennent en outre un troisième pilier et un quatrième pilier, placé entre ladite plaque d'accueil (13) de nest (1) et ladite plaque supérieure (14), au niveau de l'extrémité proximale (P1) dudit socle (9).

4. Outil à main de manipulation de seringues selon la revendication 3, dans lequel les moyens de levage (22) comprennent deux mécanismes en ciseaux de part et d'autre du socle, chaque mécanisme en ciseaux comportant deux bras de ciseaux (27,28) montés de manière pivotante autour d'un point de pivot (29) sensiblement central, le premier bras de ciseaux (27) étant relié à une extrémité, à un coin (30) de la plaque supérieure (14) de manière pivotante et à l'autre extrémité à la base (11) dudit socle (9), du côté de l'extrémité proximale (P1) de celui-ci, de manière coulissante via un premier moyen de coulissement (31), le deuxième bras de ciseaux (28) étant relié à une extrémité, à la base (11)dudit socle (9), du côté de l'extrémité distale (D1), à un coin (32), de manière pivotante et à l'autre extrémité, à la plaque supérieure (14), du côté de l'extrémité proximale (P1) du socle (9), de manière coulissante via un deuxième moyen de coulissement (33).

5. Outil à main de manipulation de seringues selon la revendication 4, dans lequel le premier moyen de coulissement comprend une gorge (34) placée sur ladite base (11) dans laquelle un élément mobile (31) relié au premier bras de ciseau (27) se déplace, ou un rail (34) muni d'orifices (35) sous forme de trous ou d'encoches, placé sur ladite base (11) dans lequel un élément mobile (31) comportant une protubérance complémentaire auxdits orifices, relié au premier bras de ciseau (27) se déplace.

6. Outil à main de manipulation de seringues selon la revendication 4 ou 5, dans lequel le deuxième moyen de coulissement comprend une gorge (36) placée sur ladite plaque supérieure (14) dans laquelle un élément mobile (33) relié au deuxième bras de ciseau (28) se déplace, ou un rail (36) muni d'orifices (37) sous forme de trous ou d'encoches, placé sur ladite plaque supérieure (14) dans lequel un élément mobile (33) comportant une protubérance complémentaire auxdits orifices, relié au deuxième bras de ciseau (28) se déplace.

7. Outil à main de manipulation de seringues selon l'une quelconque des revendications précédentes, dans lequel la plaque supérieure (14) comporte latéralement, de chaque côté, un épaulement longitudinal (38,39) comprenant une paroi horizontale (38) et une paroi s'étendant vers le haut (39), à partir de ladite paroi horizontale (38), agencé pour guider le peigne mobile (15) pendant son déplacement, et éventuellement un épaulement transversal (40,41) au niveau de l'extrémité distale (D1) de la plaque supérieure (14), comprenant une paroi horizontale (40) et une paroi s'étendant vers le haut (41), à partir de ladite paroi horizontale (40), agencée pour recevoir en butée ledit peigne mobile (15) pendant son déplacement et éventuellement des moyens de centrage (42,43) dudit peigne mobile (15), comme par exemple, deux bavettes latérales (42) et une bavette distale (43).

8. Outil à main de manipulation de seringues selon l'une quelconque des revendications précédentes, dans lequel ledit peigne mobile (15) comprend des moyens d'obturation (44) desdits x espaces interdentaires (21), situé au niveau de l'extrémité proximale (P2) dudit peigne mobile (15), lesdits moyens d'obturation (44) présentant une position d'ouverture selon laquelle lesdits espaces interdentaires (21) sont accessibles et une position de fermeture selon laquelle lesdits espaces interdentaires (21) sont fermés, et éventuellement des moyens d'actionnement (45) desdits moyens d'obturation (44), agencés pour permettre le passage desdits moyens d'obturation (44) de la position d'ouverture à la position de fermeture, ou de la position de fermeture à la position d'ouverture, éventuellement positionnés sur la poignée (19).

9. Outil à main de manipulation de seringues selon l'une quelconque des revendications précédentes, dans lequel ledit peigne mobile (15) comprend des espaces interdentaires (21) pairs agencés pour loger chacun une série de n seringues (2) et former des rangées paires de n seringues et des espaces interdentaires (21) impairs agencés pour loger chacun une série de n seringues (2) et former des rangées impaires de n seringues (2), et comprend des moyens de centrage (46) agencés pour décaler les rangées paires de n seringues des rangées impaires de n seringues.

10. Procédé de dénestage de seringues à l'aide d'un outil à main de manipulation de seringues comprenant les étapes suivantes :
- Une fourniture d'un nest (1) comprenant des orifices agencés pour loger des seringues (2) comprenant chacun un col (4) s'étendant vers le haut, des seringues (2) munies de collerettes de corps de seringues (2) , leurs aiguilles (5) et de leur capuchons d'aiguille (6), les collerettes de corps de seringues (2) reposant sur l'extrémité supérieure desdits cols (4),
- Un placement du nest (1), dans un socle (9) comprenant une plaque d'accueil (13) de nest (1), dans un orifice de celle-ci avec les rebords du nest (1) qui reposent sur la plaque d'accueil (13) de nest (1) et les capuchons d'aiguilles (6) qui reposent sur une base (11, 11b) dudit socle (9), lesdites collerettes étant en outre relevées et espacées d'une distance hA mesurée entre une face inférieure de ladite collerette et l'arête supérieure dudit col (4),
- Une préhension d'un peigne mobile (15) comprenant une poignée (19) et x-1 dents de séparation (20) d'une épaisseur hB et x espaces interdentaires (21), lesdits espaces interdentaires (21) étant agencés pour loger des seringues (2), de préférence n seringues (2), dans une position selon laquelle des collerettes de corps de seringues (2) reposent sur une face supérieure desdites dents de séparation (20),
- Une amenée du peigne mobile (15) par déplacement sur une plaque supérieure (14) dudit socle (9) présentant une position basse et une position haute,
- Un premier glissement du peigne mobile (15) sous les collerettes des seringues, durant lequel la plaque supérieure (14) est en position basse et sur laquelle se déplace le peigne mobile (15) , jusqu'à loger dans les espaces interdentaires (21) les seringues (2),
- Un levage de ladite plaque supérieure (14) jusqu'à sa position haute,
- Un deuxième glissement du peigne mobile (15) dans le sens inverse dudit premier glissement, ledit peigne (15) comportant dans les espaces interdentaires (21) les seringues (2), et un retrait du peigne mobile (15) agencé pour déplacer les seringues (2) vers une destination voulue.

11. Procédé de dénestage de seringues (2) à l'aide d'un outil à main de manipulation de seringues (2) selon la revendication 10, dans lequel une obturation des espaces interdentaires a lieu entre le levage et le deuxième glissement ou entre le premier glissement et le levage par actionnement de moyens d'obturation (44) des espaces interdentaires (21).

12. Procédé de dénestage de seringues (2) à l'aide d'un outil à main de manipulation de seringues (2) selon la revendication 10 ou la revendication 11, comprenant un positionnement de la plaque supérieure (14) en position basse avant le placement du peigne mobile (15), ou avant l'amenée du peigne mobile (15).

13. Procédé de remise en nest de seringues (2) à l'aide d'un outil à main de manipulation de seringues (2) comprenant les étapes de :
- Une fourniture d'un nest (1) ou d'un tub (7) comprenant un nest (1) comprenant des orifices agencés pour loger des seringues (2) comprenant chacun un col (4) s'étendant vers le haut,
- Un placement dudit nest (1) ou tub (7) comprenant le nest (1) dans un socle (9) comprenant une plaque d'accueil (13) de nest (1) , dans un orifice de celle-ci avec les rebords du nest (1) ou du tub (7) qui comprend le nest (1) qui reposent sur la plaque d'accueil (13) de nest (1),
- Une préhension d'un peigne mobile (15° comprenant une poignée (19) et x-1 dents de séparation (20) et x espaces interdentaires (21), lesdits espaces interdentaires (21) logeant des seringues (2), de préférence n seringues (2), dans une position selon laquelle des collerettes de corps de seringues (2) reposent sur une face supérieure desdites dents de séparation (2à),
- Une amenée du peigne mobile (15) par déplacement sur une plaque supérieure (14) dudit socle (9) présentant une position basse et une position haute,
- Un placement du peigne mobile (15) sur la plaque supérieure (14) en position haute,
- Un passage de ladite plaque supérieure (14) de sa position haute à la position basse,
- Un glissement du peigne mobile (15) vers l'extrémité proximale (P1) dudit socle sur ladite plaque supérieure (14) et sous les collerettes des seringues (2) durant lequel les seringues (2) sont positionnées dans les cols (4) et extraites dudit peigne mobile (15), et un retrait du peigne mobile (15).

14. Procédé de remise en nest de seringues (2) à l'aide d'un outil à main de manipulation de seringues selon la revendication 13, dans lequel un positionnement de la plaque supérieure (14) en position haute est effectué avant le placement du peigne mobile (15), ou avant l'amenée du peigne mobile (15).

15. Procédé de remise en nest de seringues (2) à l'aide d'un outil à main de manipulation de seringues selon la revendication 13 ou la revendication 14, dans lequel les espaces interdentaires (21) sont obturés durant la préhension du peigne mobile (15) et libérés avant le glissement du peigne mobile (15), comme par exemple durant l'une des étapes parmi l'amenée du peigne mobile (15), le placement, le passage de ladite plaque supérieure (14) de sa position haute à la position basse ou encore juste avant le glissement, mais après le passage de ladite plaque supérieure (14) de sa position haute à la position basse par actionnement de moyens d'obturation (44) des espaces interdentaires.

16. Procédé de remise en nest de seringues (2) à l'aide d'un outil à main de manipulation de seringues selon l'une quelconque des revendications 13 à 15, comprenant préalablement audit glissement, un déplacement des seringues (2) des rangées paires présentes dans des espaces interdentaires (21) pairs par rapport aux seringues (2) des rangées impaires présentes dans des espaces interdentaires (21) impairs, de manière à former des rangées de seringues (2) en quinconce les unes par rapport aux autres.
